(19)

Europäisches
Patentamt
European
Patent Office
Office européen
des brevets

(11) **EP 3 636 157 A1**

(12) **EUROPEAN PATENT APPLICATION**

(43) Date of publication:
15.04.2020 Bulletin 2020/16

(21) Application number: 18199643.0

(22) Date of filing: 10.10.2018

(51) Int Cl.:
A61B 6/03 (2006.01)    A61B 6/00 (2006.01)
G06T 11/00 (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• KOEHLER, Thomas
5656 AE Eindhoven (NL)
• PROKSA, Roland
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **APPARATUS FOR MULTI MATERIAL DECOMPOSITION**

(57)      The present invention relates to an apparatus (10) for X-ray dual energy data processing. It is described to provide (210) a processing unit (30) with at least one first emission spectrum characteristic of an X-ray tube operating in a first mode of operation. The processing unit is provided (220) with at least one second emission spectrum characteristic of the X-ray tube operating in a second mode of operation. The at least one first emission spectrum is different to the at least one second emission spectrum. The processing unit is provided (230) with at least two images acquired by a CT scanner acquiring interleaved projection image data where an X-ray tube is operating back and forth from the first mode of operation to the second mode of operation. A first image of the at least two images was captured by a detector of the CT scanner from X-rays produced by the X-ray tube operating in the first mode of operation and a second image of the at least two images was captured by the detector from X-rays produced by the X-ray tube operating in the second mode of operation. The processing unit is provided (240) with an afterglow function that models afterglow of the detector. The processing unit determines (250) two basis function images, the determination comprising utilizing the first image, the second image, the at least one first emission spectrum, the at least one second emission spectrum and the afterglow function.

FIG. 4

EP 3 636 157 A1

**Description**

FIELD OF THE INVENTION

The present invention relates to an apparatus for X-ray dual energy data processing, to a system for dual energy imaging of a body part, to a method for X-ray dual energy data processing, as well as to a computer program element and a computer readable medium.

BACKGROUND OF THE INVENTION

The general background of this invention is the field of X-ray spectral computed tomography (CT). In a CT system an X-ray source emits X-ray radiation. The emitted radiation traverses an examination region with a subject or object located within and is detected by a detector array opposite the X-ray source. The detector array detects the radiation traversing the examination region and the subject and generates projection data, e.g. raw detector data or projection images. A reconstructor processes the projection data and reconstructs a volumetric image of the subject or object. X-ray Spectral CT is an imaging modality that extends the capabilities of a conventional CT system. Dual-Energy (DE) CT, which is a specific configuration of spectral CT, utilizes two attenuation values acquired simultaneously at two photon energies to solve the photoelectric and Compton contribution that consists of the mass attenuation coefficient of a material, and thus to identify an unknown material by its value of photoelectric and Compton contribution. Because any two linearly independent sums of two basis functions span the entire attenuation coefficient space, any material can be represented by a linear combination of two other materials, so called basis materials, such as water and iodine. The basis material images provide new applications such as monochromatic image, material cancellation image, effective atomic number image and electron density image. The principle of the processing goes back to Alvarez and Macovski in the 1970s: R.E. Alvarez and A. Macovski, "Energy-selective Reconstructions in X-ray Computerized Tomography", Phys. Med. Biol., 1976, Vol. 21. No. 5, 733-744.

There are several approaches to perform dual energy CT acquisition such as fast kVp (Kilovolt-Peak) switching, and dual-layer detector configurations. Detector based dual energy CT has several advantages for dual energy imaging, namely it is always on, and it does not compromise on spatial resolution or field of view. However, a major shortcoming of detector based dual energy CT is the high hardware cost. However, for fast kVp switching an issue is afterglow of the detector. The detector is used to measure quantitatively the X-ray intensity that passed the object. Currently, the most widely used detector concept consists of a scintillator that converts x-ray to optical photons, a photo-diode that converts the optical photons into an electrical current, and some circuitry that integrates the current and converts it to a digital signal. Afterglow is a feature of the scintillator that comprises a material that exhibits scintillation - a property of luminescence - when excited by ionizing radiation. Afterglow leads to a signal leakage from each reading to the following one. Whilst this leads in conventional CT typically to just a minor loss of angular resolution, it can have a significant impact on the material decomposition in DE CT, since subsequent readings are measured with different tube spectra.

Classical afterglow correction algorithms use de-convolution type approaches to estimate and subtract the afterglow content in each reading, assuming a constant x-ray spectrum of subsequent readings. However, these cannot be applied to acquisitions with kVp switching in a straight-forward manner.

There is a need to address these issues.

SUMMARY OF THE INVENTION

It would be advantageous to have improved apparatus for X-ray dual energy data processing.

The object of the present invention is solved with the subject matter of the independent claims, wherein further embodiments are incorporated in the dependent claims. It should be noted that the following described aspects and examples of the invention apply also to the apparatus for X-ray dual energy data processing, the system for dual energy imaging of a body part, the method for X-ray dual energy data processing and for the computer program element and the computer readable medium.

According to a first aspect, there is provided an apparatus for CT X-ray dual energy data processing, comprising:

- an input unit; and
- a processing unit.

The input unit is configured to provide the processing unit with at least one first emission spectrum. The at least one first emission spectrum is characteristic of an X-ray tube operating in a first mode of operation. The input unit is configured also to provide the processing unit with at least one second emission spectrum. The at least one second emission spectrum is characteristic of the X-ray tube operating in a second mode of operation. The at least one first emission spectrum is different to the at least one second emission spectrum. The input unit is configured also to provide the processing unit with at least two images acquired by a CT X-ray scanner acquiring interleaved projection image data. An X-ray tube of the CT scanner is operating back and forth between the first mode of operation and the second mode of operation. A first image of the at least two images was acquired by a detector of the CT scanner from X-rays produced by the X-ray tube operating in the first mode of operation. A second image of the at least two images was acquired by the detector from X-rays produced by the X-ray tube operating in the second mode of operation. The input unit is configured also to provide the processing unit with an afterglow function that models afterglow of the detector. The processing unit is configured to determine two basis function images, the determination comprising utilization of the first image, the second image, the at least one first emission spectrum, the at least one second emission spectrum and the afterglow function.

In other words, afterglow correction can be performed through utilization of X-ray tube emission spectra and an afterglow function that models afterglow, enabling standard dual energy processing algorithms to be used to provide for material decomposition from images acquired by fast switching based system.

Thus, characteristic or reference spectra for an X-ray tube that is operating in two modes of operation that produces different emission spectra in those modes, such as for a fast switching kVp system of for an X-ray tube where a spectrum altering filter is inserted in one mode of operation and extracted for the other mode of operation, as used. These characteristic spectra, that could be calculated from first principles or experimentally determined under steady state conditions, are then used in combination with CT X-ray data acquired by a system that is now operating in the first mode and then in the second mode, for example a fast kVp switching system or the above mentioned selective filtering. Normally, afterglow in the detector means that an image acquired with respect to the X-ray tube generating a certain emission spectrum or spectra also contains some signal from the immediately previous operation of the X-ray tube generating a different emission spectrum or spectrum that then leads to errors in material decomposition. But now, an afterglow function is used with the characteristic emission spectra and the image data to enable the effects of afterglow to be accounted for when performing material decomposition.

In an example, the determination of the two basis function images comprises at least one first convolution comprising utilization of the at least one first emission spectrum with the afterglow function and comprises at least one second convolution comprising utilization of the at least one first emission spectrum with the afterglow function.

Thus, standard dual energy processing algorithms can be used for material decomposition, where now emission spectra from an X-ray tube are convolved with the afterglow function.

In an example, the first mode of operation relates to the X-ray tube operating over at least one first voltage, and the second mode of operation relates to the X-ray tube operating over at least one second voltage. The input unit is configured to provide the processing unit with a plurality of first X-ray tube voltage values at different times for the at least one first voltage and the at least one first emission spectrum are a plurality of first emission spectra characteristic of the X-ray tube operating at the plurality of first X-ray tube voltages. The input unit is configured also to provide the processing unit with a plurality of second X-ray tube voltage values at different times for the at least one second voltage and the at least one second emission spectrum are a plurality of second emission spectra characteristic of the X-ray tube operating at the plurality of second X-ray voltages.

Thus, as the X-ray tube transitions from low to high voltages and back again in a fast switching arrangement, the tube voltages continuously transitions from these voltages, and the emission spectra from the X-ray tube also continuous varies. Thus, a convolution of the time-dependent spectra is performed with a known afterglow function that models the afterglow of the X-ray tube and mean spectra are calculated for the two integration periods of the detector.

In an example, the at least one first emission spectrum and the at least one second emission spectrum are calculated from first principles.

In an example, the at least one first emission spectrum and the at least one second emission spectrum are experimentally determined.

In an example, the afterglow function is a low pass kernel that models the afterglow.

According to a second aspect, there is provided a medical system for dual energy imaging of a body part, the system comprising:

- a CT X-ray scanner configured to acquire interleaved sets of projection image data;
- an apparatus for dual energy data processing according to the first aspect; and
- an output unit.

The CT X-ray scanner comprises an X-ray tube and a detector. The X-ray tube is configured to operate back and forth between the first mode of operation and the second mode of operation. The detector is configured to acquire a first image of a body part generated from X-rays produced by the X-ray tube operating in the first mode of operation, and acquire a second image of the body part generated from X-rays produced by the X-ray tube operating in the second mode of operation. The apparatus is configured to determine two basis function images of the body part. The output unit is configured to output the two basis function images of the body part.

In an example, the first mode of operation relates to the X-ray tube operating over at least one first voltage, and the second mode of operation relates to the X-ray tube operating over at least one second voltage. The image acquisition unit comprises a voltage measurement unit configured to measure voltages of the X-ray tube. The voltage measurement unit is configured also to provide the apparatus with a plurality of first X-ray tube voltage values at different times for the at least one first voltage and the at least one first emission spectrum are a plurality of first emission spectra characteristic of the X-ray tube operating at the plurality of first X-ray tube voltages. The voltage measurement unit is configured also to provide the apparatus with a plurality of second X-ray tube voltage values at different times for the at least one second voltage and the at least one second emission spectrum are a plurality of second emission spectra characteristic of the X-ray tube operating at the plurality of second X-ray voltages.

In an example, the voltage measurement unit is configured to measure the X-ray tube voltage at 100 kHz.

According to a third aspect, there is provided a method for X-ray dual energy data processing, comprising:

a) providing a processing unit with at least one first emission spectrum characteristic of an X-ray tube operating in a first mode of operation;
b) providing the processing unit with at least one second emission spectrum characteristic of the X-ray tube operating in a second mode of operation, wherein the at least one first emission spectrum is different to the at least one second emission spectrum;
c) providing the processing unit with at least two images acquired by a CT scanner acquiring interleaved projection image data where an X-ray tube is operating back and forth from the first mode of operation to the second mode of operation; wherein a first image of the at least two images was captured by a detector of the CT scanner from X-rays produced by the X-ray tube operating in the first mode of operation and a second image of the at least two images was captured by the detector from X-rays produced by the X-ray tube operating in the second mode of operation;
d) providing the processing unit with an afterglow function that models afterglow of the detector; and
e) determining by the processing unit two basis function images, the determination comprising utilizing the first image, the second image, the at least one first emission spectrum, the at least one second emission spectrum and the afterglow function.

In an example, step e) comprises at least one first convolution comprising utilizing the at least one first emission spectrum with the afterglow function and comprises at least one second convolution comprising utilizing the at least one first emission spectrum with the afterglow function.

In an example, the first mode of operation relates to the X-ray tube operating over at least one first voltage, and the second mode of operation relates to the X-ray tube operating over at least one second voltage. The method then also comprises providing the processing unit with a plurality of first X-ray tube voltage values at different times for the at least one first voltage and the at least one first emission spectrum are a plurality of first emission spectra characteristic of the X-ray tube operating at the plurality of first X-ray tube voltages. The method also comprises providing the processing unit with a plurality of second X-ray tube voltage values at different times for the at least one second voltage and the at least one second emission spectrum are a plurality of second emission spectra characteristic of the X-ray tube operating at the plurality of second X-ray voltages.

In an example, the method comprises measuring the X-ray tube voltage by a voltage measurement unit to generate the first X-ray tube voltage values and the second X-ray tube voltage values.

According to another aspect, there is provided a computer program element controlling apparatus as previously described which, in the computer program element is executed by processing unit, is adapted to perform the method steps as previously described. According to another aspect, there is provided a computer readable medium having stored computer element as previously described.

Advantageously, the benefits provided by any of the above aspects equally apply to all of the other aspects and vice versa.

The above aspects and examples will become apparent from and be elucidated with reference to the embodiments described hereinafter.

BRIEF DESCRIPTION OF THE DRAWINGS

Exemplary embodiments will be described in the following with reference to the following drawings:

Fig. 1 shows a schematic set up of an example of an apparatus for X-ray dual energy data processing;
Fig. 2 shows a schematic set up of an example of a system for dual energy imaging of a body part;
Fig. 3 shows a method for X-ray dual energy data processing;

Fig. 4 shows a schematic example voltage waveform for an X-ray tube used for fast kVp switching;
Fig. 5 shows an example of the effects of afterglow; and
Fig. 6 shows an example of the effects of afterglow before and after X-ray dual energy data processing.

DETAILED DESCRIPTION OF EMBODIMENTS

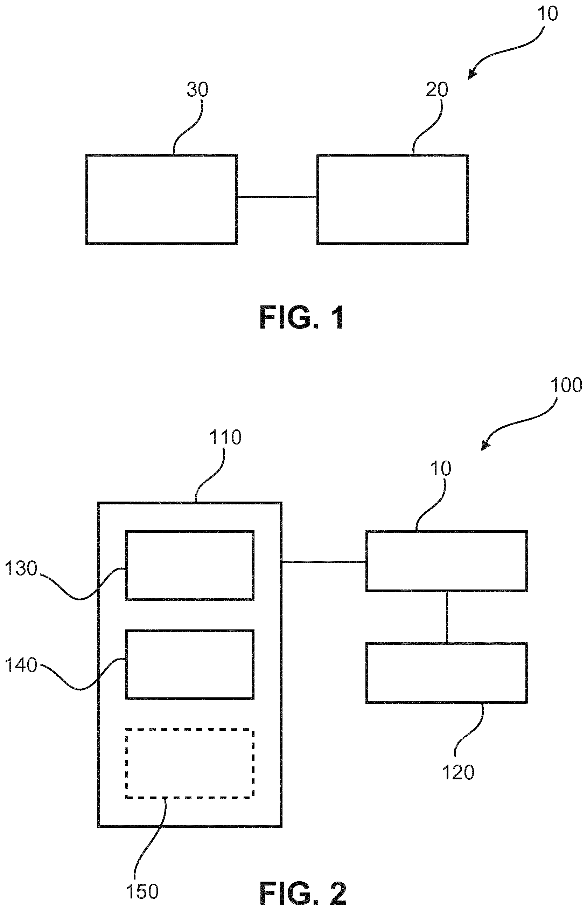

Fig. 1 shows an example of an apparatus 10 for CT X-ray dual energy data processing. The apparatus comprises an input unit 20, and a processing unit 30. The input unit is configured to provide the processing unit with at least one first emission spectrum. The at least one first emission spectrum is characteristic of an X-ray tube operating in a first mode of operation. The input unit is configured also to provide the processing unit with at least one second emission spectrum. The at least one second emission spectrum is characteristic of the X-ray tube operating in a second mode of operation. The at least one first emission spectrum is different to the at least one second emission spectrum. The input unit is configured also to provide the processing unit with at least two images acquired by a CT X-ray scanner acquiring interleaved projection image data. An X-ray tube of the CT scanner is (or has been) operating back and forth between the first mode of operation and the second mode of operation. A first image of the at least two images was acquired by a detector of the CT scanner from X-rays produced by the X-ray tube operating in the first mode of operation. A second image of the at least two images was acquired by the detector from X-rays produced by the X-ray tube operating in the second mode of operation. The input unit is configured also to provide the processing unit with an afterglow function that models afterglow of the detector. The processing unit is configured to determine two basis function images, the determination comprising utilization of the first image, the second image, the at least one first emission spectrum, the at least one second emission spectrum and the afterglow function.

In an example, the two basis function images are a photoelectric scattering image and a Compton scattering image.

In an example, the two basis function images are a water image and an Iodine image.

In an example, the two basis function images are a water image and a Calcium image.

In an example, the two basis function images are a PMMA image and an Aluminium image.

In other words, a decomposed set of projection data are calculated using the two sets of images, the two emission spectra and the afterglow function, where decomposition means that projection data of material basis functions are generated. The materials can be "abstract" photo-electric and Compton scattering, or real materials like water and Calcium, PMMA and Al, or the like.

In an example, the first mode of operation relates to the X-ray tube operating over at least one first voltage.

In an example, the second mode of operation relates to the X-ray tube operating over at least one second voltage.

In an example, the at least one second voltage is greater than the at least one first voltage.

According to an example, the determination of the two basis function images comprises at least one first convolution comprising utilization of the at least one first emission spectrum with the afterglow function. The determination of the two basis function images comprises also at least one second convolution comprising utilization of the at least one first emission spectrum with the afterglow function.

According to an example, the first mode of operation relates to the X-ray tube operating over at least one first voltage, and the second mode of operation relates to the X-ray tube operating over at least one second voltage. The input unit is configured to provide the processing unit with a plurality of first X-ray tube voltage values at different times for the at least one first voltage. The at least one first emission spectrum are a plurality of first emission spectra characteristic of the X-ray tube operating at the plurality of first X-ray tube voltages. The input unit is configured also to provide the processing unit with a plurality of second X-ray tube voltage values at different times for the at least one second voltage. The at least one second emission spectrum are a plurality of second emission spectra characteristic of the X-ray tube operating at the plurality of second X-ray voltages.

According to an example, the at least one first emission spectrum and the at least one second emission spectrum are calculated from first principles.

According to an example, the at least one first emission spectrum and the at least one second emission spectrum are experimentally determined.

According to an example, the afterglow function is a low pass kernel that models the afterglow.

Fig. 2 shows an example of a medical system 100 for dual energy imaging of a body part. The system comprises a CT X-ray scanner 110 configured to acquire interleaved sets of projection image data. The system also comprises an apparatus 10 for dual energy data processing as described with reference to Fig. 1. The system also comprises an output unit 120. The CT X-ray scanner comprises an X-ray tube 130 and a detector 140. The X-ray tube is configured to operate back and forth between the first mode of operation and the second mode of operation. The detector is configured to acquire a first image of a body part generated from X-rays produced by the X-ray tube operating in the first mode of operation, and acquire a second image of the body part generated from X-rays produced by the X-ray tube operating in the second mode of operation. The apparatus is configured to determine two basis function images of the body part. The output unit is configured to output the two basis function images of the body part.

According to an example, the first mode of operation relates to the X-ray tube operating over at least one first voltage, and the second mode of operation relates to the X-ray tube operating over at least one second voltage. The image acquisition unit comprises a voltage measurement unit 150 configured to measure voltages of the X-ray tube. The voltage measurement unit is configured to provide the apparatus with a plurality of first X-ray tube voltage values at different times for the at least one first voltage. The at least one first emission spectrum are a plurality of first emission spectra characteristic of the X-ray tube operating at the plurality of first X-ray tube voltages. The voltage measurement unit is configured also to provide the apparatus with a plurality of second X-ray tube voltage values at different times for the at least one second voltage. The at least one second emission spectrum are a plurality of second emission spectra characteristic of the X-ray tube operating at the plurality of second X-ray voltages.

According to an example, the voltage measurement unit is configured to measure the X-ray tube voltage at 100 kHz.

In different examples, the voltage measurement units can operate at 10kHz, 20kHz, 50kHz, 75kHz, 150kHz, 200kHz.

Rather than use a measurement unit, in another example voltage waveforms are calibrated and stored in some look-up tables. Then the input unit is configured to provide the processing unit with the appropriate, correct, table.

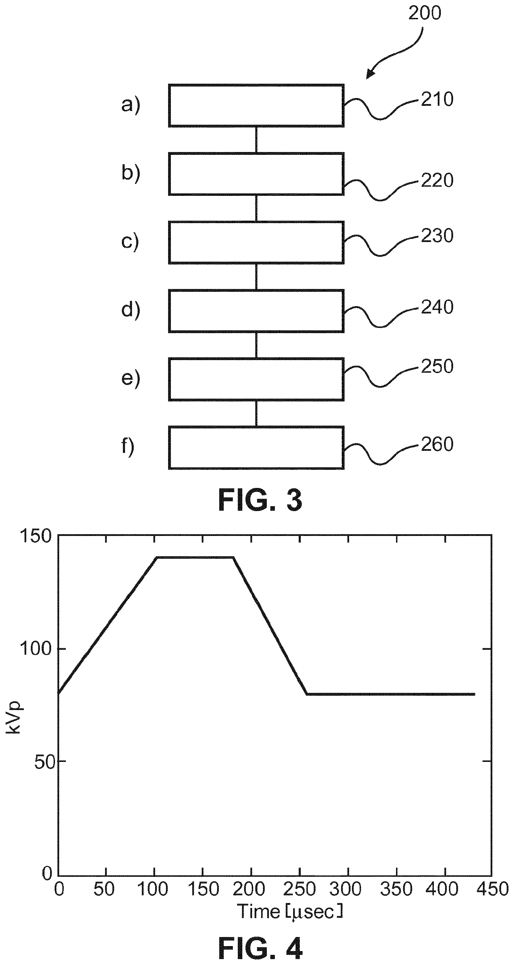

Fig. 3 shows a method 200 for X-ray dual energy data processing in its basic steps. The method comprises:

in a providing step 210, also referred to as step a), providing a processing unit 30 with at least one first emission spectrum characteristic of an X-ray tube operating in a first mode of operation;

in a providing step 220, also referred to as step b), providing the processing unit with at least one second emission spectrum characteristic of the X-ray tube operating in a second mode of operation, wherein the at least one first emission spectrum is different to the at least one second emission spectrum;

in a providing step 230, also referred to as step c), providing the processing unit with at least two images acquired by a CT scanner acquiring interleaved projection image data, wherein an X-ray tube is operating back and forth from the first mode of operation to the second mode of operation. A first image of the at least two images was captured by a detector of the CT scanner from X-rays produced by the X-ray tube operating in the first mode of operation and a second image of the at least two images was captured by the detector from X-rays produced by the X-ray tube operating in the second mode of operation;

in a providing step 240, also referred to as step d), providing the processing unit with an afterglow function that models afterglow of the detector; and

in a determining step 250, also referred to as step e), determining by the processing unit two basis function images, the determination comprising utilizing the first image, the second image, the at least one first emission spectrum, the at least one second emission spectrum and the afterglow function.

According to an example, step e) comprises at least one first convolution comprising utilizing the at least one first emission spectrum with the afterglow function and comprises at least one second convolution comprising utilizing the at least one first emission spectrum with the afterglow function.

According to an example, the first mode of operation relates to the X-ray tube operating over at least one first voltage, and the second mode of operation relates to the X-ray tube operating over at least one second voltage. The method then comprises providing the processing unit with a plurality of first X-ray tube voltage values at different times for the at least one first voltage and the at least one first emission spectrum are a plurality of first emission spectra characteristic of the X-ray tube operating at the plurality of first X-ray tube voltages. The method then comprises also providing the processing unit with a plurality of second X-ray tube voltage values at different times for the at least one second voltage and the at least one second emission spectrum are a plurality of second emission spectra characteristic of the X-ray tube operating at the plurality of second X-ray voltages.

According to an example, the method comprises measuring the X-ray tube voltage by a voltage measurement unit to generate the first X-ray tube voltage values and the second X-ray tube voltage values.

In an example, the voltage measurement unit is operating at 100kHz.

In an example, the at least one first emission spectrum and the at least one second emission spectrum are calculated from first principles.

In an example, the at least one first emission spectrum and the at least one second emission spectrum are experimentally determined.

In an example, the afterglow function is a low pass kernel that models the afterglow.

In an example, the afterglow function is a single sided exponential decay function.

The apparatus for X-ray dual energy data processing, system for dual energy imaging of a body part, and method for X-ray dual energy data processing will now additionally be described in conjunction with Figs. 4-6.

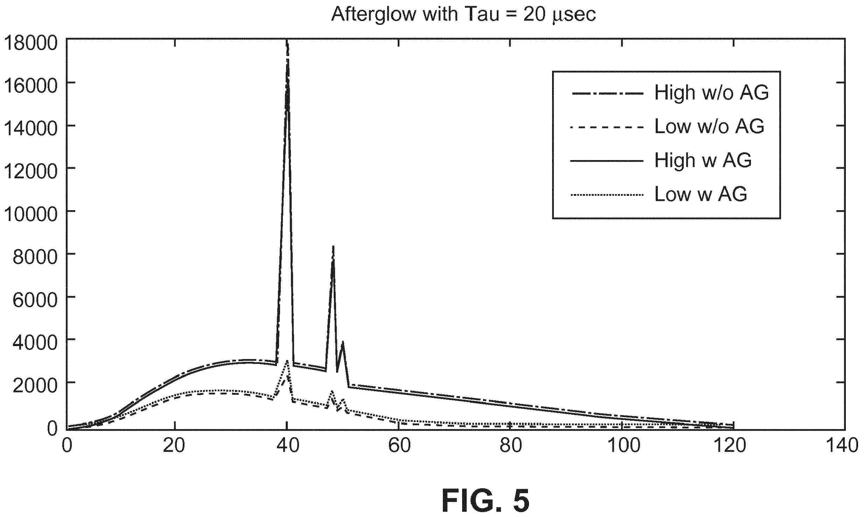

Fig. 4 is a schematic representation of the voltage of the X-ray tube used in fast kVp switching. X-ray are generated as the X-ray tube voltage ramps up and ramps down, and back again. At each voltage, the X-rays have characteristic spectra that are a function of the voltage. The X-rays pass through an examination region and are detected by a detector that in effect integrates the signal over a first integration period when the X-ray tube is at a "high" voltage and over a second integration period when the X-ray tube is at a "low" voltage. However, as discussed above the detector suffers from afterglow that leads to some signal when the X-ray tube was operating at the High voltage, that without afterglow would have contributed to the integrated High signal, but with afterglow leaks into the integrated Low signal, and vice versa. This situation is shown in Fig. 5.

The solution is to model the effect of afterglow as a modification of the effective spectra. Let's denote the tube voltage waveform as $u(t)$, the tube's emission spectrum as $T(E, u(t))$, and the system filtration, such as a Bowtie filter/window etc., by $F(E)$.

For the modelling, the case without afterglow is first considered and it is assumed that the attenuation by the patient does not change during one integration period (IP). Then, the energy dependent attenuation by the patient can be written as $P(E) = e^{-\int ds\, \mu(s, E)}$.

Using this, the scintillator input spectrum can be written as

$$G_{In}(E, t) = T\big(E, u(t)\big) F(E) P(E)$$

and the scintillator output signal becomes

$$G_{Out}(t) \propto \int E G_{In}(E, t)\, dE$$

The electronics integrates the signal for one IP (from t0... t1) and generates the reading

$$R \propto \int_{t_0}^{t_1} G_{Out}(t)\, dt = \int_{t_0}^{t_1} dt \int E F(E) P(E) T(E, u(t))\, dE$$

Now the case with afterglow is considered. Afterglow will convolve the scintillator output

$$G_{Out}(t) \propto \int E G_{In}(E, t)\, dE$$

with $a(t)$, a low pass kernel that models the afterglow (an afterglow function), and becomes

$$\tilde{G}_{Out}(t) \propto \int a(\tau)\, d\tau \int E G_{In}(E, t - \tau)\, dE$$

and the detector reading becomes

$$\tilde{R} \propto \int_{t_0}^{t_1} dt \int a(\tau)\, d\tau \int E T\big(E, u(t - \tau)\big) F(E) P(E)\, dE$$

exchanging the order of integration yields

$$\tilde{R} \propto \int_{t_0}^{t_1} dt \int E F(E) P(E)\, dE \int a(\tau) T\big(E, u(t - \tau)\big)\, d\tau$$

which is equivalent to

$$\tilde{R} \propto \int_{t_0}^{t_1} dt \int EF(E)P(E)\tilde{T}(E,t)dE$$

with

$$\tilde{T}(E,t) = \int a(\tau)T\big(E, u(t-\tau)\big)d\tau$$

This result indicates that afterglow correction can be performed by standard dual energy processing algorithms. The only change to be done is to perform a convolution of the time-dependent emission spectrum with the low pass kernel that models the afterglow.

In a specific embodiment, an appropriate system, such as a generator or input unit, provides $T(E, t)$, i.e. the tube spectrum as a function of time and provides the afterglow function $a(t)$. It can also provide the filtration $F(E)$, but this can be considered to be a part of $T$.

The afterglow function is typically a single sided exponential decay, i.e.

$$a(t) = \begin{cases} 0, & t < 0 \\ e^{-\tau t} & t \geq 0 \end{cases}$$

Therefore, $\tilde{T}$ can be calculated numerically, i.e. by summing over discrete time points. For $a$ that is decreasing on a time-scale given by $\tau$, i.e. for $t >> \tau$, the following applies $a(t) \approx 0$. Thus, for a proper numerical evaluation of the integral, $T(E,t)$ should be provided by the system at a temporal sampling period not worse that $\tau$.

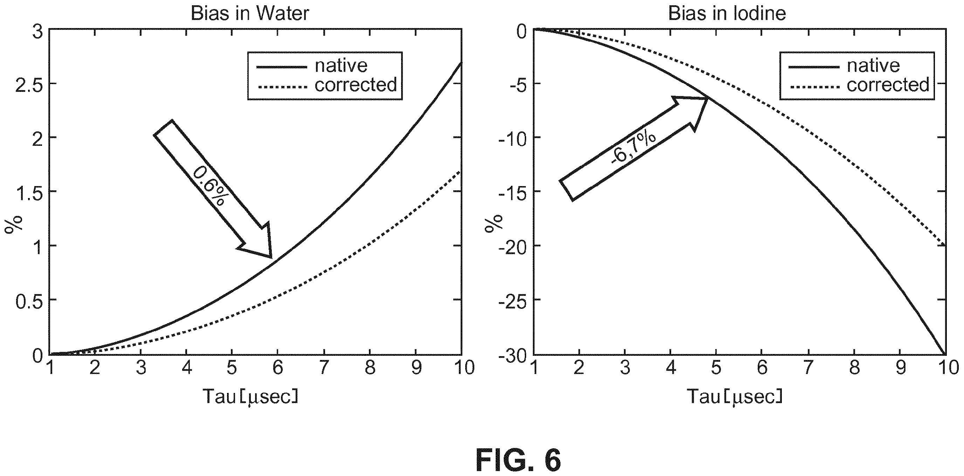

Fig. 6 shows a simulation of the afterglow induced bias in the water (left) and iodine (right) image as a function of the scintillators afterglow. This demonstrates the impact of afterglow on the imaging chain, where the bias in a water-iodine decomposition is shown as a function of the primary decay time. For 5 $\mu$s as primary decay time (which is an exemplar value for detector), the bias is as large as 0.6% in the water image and 6.7% in the iodine image. However, after correction as described above the bias is 0.3% and 4.5%, indicating that the described technique has better corrected for the effects of afterglow.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. An apparatus (10) for CT X-ray dual energy data processing, comprising:

   - an input unit (20); and
   - a processing unit (30);

   wherein, the input unit is configured to provide the processing unit with at least one first emission spectrum, wherein the at least one first emission spectrum is characteristic of an X-ray tube operating in a first mode of operation;
   wherein, the input unit is configured to provide the processing unit with at least one second emission spectrum, wherein the at least one second emission spectrum is characteristic of the X-ray tube operating in a second mode of operation, wherein the at least one first emission spectrum is different to the at least one second emission spectrum;
   wherein, the input unit is configured to provide the processing unit with at least two images acquired by a CT X-ray scanner acquiring interleaved projection image data wherein an X-ray tube of the CT scanner is operating back and forth between the first mode of operation and the second mode of operation;
   wherein a first image of the at least two images was acquired by a detector of the CT scanner from X-rays produced by the X-ray tube operating in the first mode of operation, and a second image of the at least two images was acquired by the detector from X-rays produced by the X-ray tube operating in the second mode of operation;
   wherein, the input unit is configured to provide the processing unit with an afterglow function that models afterglow of the detector; and
   wherein, the processing unit is configured to determine two basis function images, the determination comprising utilization of the first image, the second image, the at least one first emission spectrum, the at least one second emission spectrum and the afterglow function.

2. Apparatus according to claim 1, wherein the determination of the two basis function images comprises at least one first convolution comprising utilization of the at least one first emission spectrum with the afterglow function and comprises at least one second convolution comprising utilization of the at least one first emission spectrum with the afterglow function.

3. Apparatus according to any of claims 1-2, wherein the first mode of operation relates to the X-ray tube operating over at least one first voltage, and the second mode of operation relates to the X-ray tube operating over at least one second voltage; and wherein the input unit is configured to provide the processing unit with a plurality of first X-ray tube voltage values at different times for the at least one first voltage and wherein the at least one first emission spectrum are a plurality of first emission spectra characteristic of the X-ray tube operating at the plurality of first X-ray tube voltages; and wherein the input unit is configured to provide the processing unit with a plurality of second X-ray tube voltage values at different times for the at least one second voltage and wherein the at least one second emission spectrum are a plurality of second emission spectra characteristic of the X-ray tube operating at the plurality of second X-ray voltages.

4. Apparatus according to any of claims 1-3, wherein the at least one first emission spectrum and the at least one second emission spectrum are calculated from first principles.

5. Apparatus according to any of claims 1-3, wherein the at least one first emission spectrum and the at least one second emission spectrum are experimentally determined.

6. Apparatus according to any of claims 1-5, wherein the afterglow function is a low pass kernel that models the afterglow.

7. A medical system (100) for dual energy imaging of a body part, the system comprising:

   - a CT X-ray scanner (110) configured to acquire interleaved sets of projection image data;
   - an apparatus (10) for dual energy data processing according to any of claims 1-6; and
   - an output unit (120);

   wherein, the CT X-ray scanner comprises an X-ray tube (130) and a detector (140);
   wherein the X-ray tube is configured to operate back and forth between the first mode of operation and the second mode of operation;
   wherein, the detector is configured to acquire a first image of a body part generated from X-rays produced by the X-ray tube operating in the first mode of operation, and acquire a second image of the body part generated from X-rays produced by the X-ray tube operating in the second mode of operation;
   wherein, the apparatus is configured to determine two basis function images of the body part; and
   wherein, the output unit is configured to output the two basis function images of the body part.

8. Medical system according to claim 7, wherein the first mode of operation relates to the X-ray tube operating over at least one first voltage, and the second mode of operation relates to the X-ray tube operating over at least one second voltage; and wherein the image acquisition unit comprises a voltage measurement unit (150) configured to measure voltages of the X-ray tube, and configured to provide the apparatus with a plurality of first X-ray tube voltage values at different times for the at least one first voltage and wherein the at least one first emission spectrum are a plurality of first emission spectra characteristic of the X-ray tube operating at the plurality of first X-ray tube voltages; and provide the apparatus with a plurality of second X-ray tube voltage values at different times for the at least one second voltage and wherein the at least one second emission spectrum are a plurality of second emission spectra characteristic of the X-ray tube operating at the plurality of second X-ray voltages.

9. Medical system according to claim 8, wherein the voltage measurement unit is configured to measure the X-ray tube voltage at 100 kHz.

10. A method (200) for X-ray dual energy data processing, comprising:

   a) providing (210) a processing unit (30) with at least one first emission spectrum characteristic of an X-ray tube operating in a first mode of operation;
   b) providing (220) the processing unit with at least one second emission spectrum characteristic of the X-ray tube operating in a second mode of operation, wherein the at least one first emission spectrum is different to the at least one second emission spectrum;
   c) providing (230) the processing unit with at least two images acquired by a CT scanner acquiring interleaved projection image data where an X-ray tube is operating back and forth from the first mode of operation to the second mode of operation; wherein a first image of the at least two images was captured by a detector of the CT scanner from X-rays produced by the X-ray tube operating in the first mode of operation and a second image of the at least two images was captured by the detector from X-rays produced by the X-ray tube operating in the second mode of operation;
   d) providing (240) the processing unit with an afterglow function that models afterglow of the detector; and
   e) determining (250) by the processing unit two basis function images, the determination comprising utilizing the first image, the second image, the at least one first emission spectrum, the at least one second emission spectrum and the afterglow function.

11. Method according to claim 10, wherein step e) comprises at least one first convolution comprising utilizing the at least one first emission spectrum with the afterglow function and comprises at least one second convolution comprising utilizing the at least one first emission spectrum with the afterglow function.

**12.** Method according to any of claims 10-11, wherein, the first mode of operation relates to the X-ray tube operating over at least one first voltage, and the second mode of operation relates to the X-ray tube operating over at least one second voltage, and the method comprises providing the processing unit with a plurality of first X-ray tube voltage values at different times for the at least one first voltage and wherein the at least one first emission spectrum are a plurality of first emission spectra characteristic of the X-ray tube operating at the plurality of first X-ray tube voltages; and the method comprises providing the processing unit with a plurality of second X-ray tube voltage values at different times for the at least one second voltage and wherein the at least one second emission spectrum are a plurality of second emission spectra characteristic of the X-ray tube operating at the plurality of second X-ray voltages.

**13.** Method according to claim 12, wherein the method comprises measuring the X-ray tube voltage by a voltage measurement unit to generate the first X-ray tube voltage values and the second X-ray tube voltage values.

**14.** A computer program for controlling an apparatus according to one of claims 1 to 6, and/or system of any of claims 7 to 9, which when executed by a processor is configured to carry out the method of any of claims 10 to 13.

**15.** A computer readable medium having stored the program element of claim 14.

**FIG. 1**

**FIG. 2**

**FIG. 3**

**FIG. 4**

Afterglow with Tau = 20 μsec

Legend:
High w/o AG
Low w/o AG
High w AG
Low w AG

**FIG. 5**

EP 3 636 157 A1

Bias in Iodine

Bias in Water

FIG. 6

Europäisches
Patentamt

European
Patent Office

Office européen
des brevets

# EUROPEAN SEARCH REPORT

Application Number

EP 18 19 9643

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 249 123 A (GENERAL ELECTRIC COMPANY) 28 September 1993 (1993-09-28) * abstract * * column 2, line 50 - column 3, line 6 * * column 4, line 57 - column 5, line 66 * ----- | 1-15 | INV. A61B6/03 A61B6/00 G06T11/00 |
| A | CARMI R ET AL: "Resolution enhancement of X-ray CT by spatial and temporal MLEM deconvolution correction", 2004 IEEE NUCLEAR SCIENCE SYMPOSIUM CONFERENCE RECORD 16-22 OCT. 2004 ROME, ITALY, IEEE, PISCATAWAY, NJ, USA, vol. 5, 16 October 2004 (2004-10-16), pages 2765-2768, XP010819270, DOI: 10.1109/NSSMIC.2004.1466262 ISBN: 978-0-7803-8700-3 * abstract * * section "I. DECONVOLUTION METHOD" * ----- | 1-15 | |
| A | US 2017/172528 A1 (WIEDMANN UWE [US] ET AL) 22 June 2017 (2017-06-22) * abstract * * paragraph [0023] - paragraph [0038] * ----- | 1-15 | TECHNICAL FIELDS SEARCHED (IPC)  A61B G06T |

The present search report has been drawn up for all claims

| Place of search | Date of completion of the search | Examiner |
|---|---|---|
| Munich | 26 April 2019 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
### ON EUROPEAN PATENT APPLICATION NO.

EP 18 19 9643

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2019

| Patent document cited in search report | | Publication date | Patent family member(s) | Publication date |
|---|---|---|---|---|
| US 5249123 | A | 28-09-1993 | NONE | |
| US 2017172528 | A1 | 22-06-2017 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R.E. ALVAREZ ; A. MACOVSKI.** Energy-selective Reconstructions in X-ray Computerized Tomography. *Phys. Med. Biol.,* 1976, vol. 21 (5), 733-744